# EUROPÄISCHE PATENTSCHRIFT

(11) **EP 3 112 818 B1**
(45) Veröffentlichungstag und Bekanntmachung des Hinweises auf die Patenterteilung: **26.02.2020**
(21) Anmeldenummer: 15174891.0
(22) Anmeldetag: 01.07.2015
(51) Int. Cl.: G01F 1/36, G01F 1/42, A61B 5/087, A61M 16/00

(54) **ATEMSTROMSENSOR**
RESPIRATORY FLOW SENSOR
CAPTEUR DE DEBIT RESPIRATOIRE

(43) Veröffentlichungstag der Anmeldung: 04.01.2017
(73) Patentinhaber: imtmedical ag, 9470 Buchs SG (CH)
(72) Erfinder: FRIBERG, Harri, 9493 Mauren (LI); DAESCHER, Jakob, 7306 Fläsch (CH)
(74) Vertreter: Patentbüro Paul Rosenich AG

(56) Entgegenhaltungen:
- EP-A1- 0 331 773
- EP-A2- 1 236 975
- EP-A2- 2 275 783
- DE-A1- 4 034 176
- US-A- 4 989 456
- US-A1- 2007 131 279

## Beschreibung

Die Erfindung betrifft einen Atemstromsensor nach dem Oberbegriff des Anspruchs 1.

Atemstromsensoren, auch Differenzdruck-Durchflusssensoren, Durchflussmessfühler oder Fluss-Sensoren genannt, werden zwischen einem von einem Beatmungsgerät oder Anästhesiegerät ausgehenden Schlauch und einem dem Patienten zugeführten Schlauch angeordnet.

Aus der US5979247A ist ein Atemstromsensor mit einem Strömungsrohr, mit einer Blendenklappe und mit Anschlüssen bekannt. Das Strömungsrohr weist einen zylindrischen Strömungskanal und an dessen freien Enden Schlauchanschlüsse für die Schläuche auf. Die Blendenklappe ist in dem Strömungskanal angeordnet und unterteilt den Strömungskanal in einen ersten Strömungskanalteil und in einen zweiten Strömungskanalteil. Weiter weist die Blendenklappe einen Scharnierbereich und einen dem Scharnierbereich gegenüberliegenden freien Endbereich auf. Der Scharnierbereich ist von einem ersten Einschnitt und von einem zweiten Einschnitt gebildet, wobei der zweite Einschnitt zwischen dem ersten Einschnitt angeordnet ist. Die Anschlüsse münden zu beiden Seiten der Blendenklappe in den Strömungskanal und dienen der Abnahme der durch die Blendenklappe erzeugten Druckdifferenz.

Durchströmt Luft beziehungsweise der Atem des Patienten den Atemstromsensor bildet die auslenkbare Blendenklappe einen Strömungswiderstand. Ein Atemstromsensor muss für eine zuverlässige Verwendung eine ausreichende Messgenauigkeit aufweisen. Insbesondere bei einem Atemstromsensor für Kinder, der einen verhältnismässig kleinen Durchmesser des Strömungskanals aufweist, treten hohe Differenzdrücke im Atemstromsensor auf. Entsprechend haben geringe Einflüsse eine grosse Auswirkung auf die durchgeführte Messung.

Die Atemluft des Patienten beinhaltet auch Feuchtigkeit, welche während der Beatmung sich im Strömungskanal des Atemstromsensors als Flüssigkeit niederschlägt. Diese Flüssigkeit kann zu einem Messrauschen auf dem Nutzsignal führen und somit eine ungenaue Messung verursachen. Die im Atemstromsensor befindliche Flüssigkeit kann auch dazu führen, dass die Messung stark verfälscht wird, da die Charakteristik der Blendenklappe infolge eines Flüssigkeitsbeschlags sich ändert. Weiter kann infolge eines hohen Flüssigkeitsstands im Strömungskanal die Bewegungsfreiheit beziehungsweise die Auslenkbarkeit der Blendenklappe stark beeinträchtigt werden. Diese Effekte treten insbesondere dann auf, wenn sich die Flüssigkeit in einem Bereich des Strömungskanals sammelt, in dem die Blendenklappe angeordnet ist.

Es wurde nun bereits vorgeschlagen, den Atemstromsensor jeweils so zwischen den Schläuchen anzuordnen, dass dieser in einer schrägen Ausrichtung zu liegen kommt und durch die Schwerkraft bedingt Flüssigkeit abfliessen kann. Der Patient bewegt sich jedoch oftmals während der Dauer eines Beatmungsvorgangs oder während einer Anästhesie. Somit kommt der schräg angeordnete Atemstromsensor gegebenenfalls in eine Lage, in welcher eine Entwässerung des Atemstromsensors und somit eine ausreichende Messgenauigkeit nicht mehr gewährleistet ist.

Durch den Scharnierbereich der Blendenklappe, der zwei zueinander beabstandete Gelenkbereiche beziehungsweise Scharniere umfasst, ist die Blendenklappe nur eingeschränkt Torsionsbelastungen insbesondere bei hohen Atemflüssen ausgesetzt. Jedoch treten nach einer gewissen Gebrauchsdauer immer wieder Brüche in diesem Scharnierbereich auf, die ebenfalls zu ungenauen Messergebnissen führen können.

Aus der DE19617738C1 ist ein Atemstromsensor mit einem Strömungsrohr, mit einer Blendenklappe und mit Anschlüssen bekannt. Das Strömungsrohr weist einen im Querschnitt rechteckförmigen Strömungskanal und an dessen freien Enden Schlauchanschlüsse für die Schläuche auf. Die Blendenklappe ist in dem Strömungskanal angeordnet und unterteilt den Strömungskanal in einen ersten Strömungskanalteil und in einen zweiten Strömungskanalteil. Weiter weist die Blendenklappe einen Scharnierbereich und einen dem Scharnierbereich gegenüberliegenden freien Endbereich auf. Der Scharnierbereich ist von einem ersten Einschnitt und von einem zweiten Einschnitt gebildet, wobei der zweite Einschnitt zwischen dem ersten Einschnitt angeordnet ist. Die Anschlüsse münden zu beiden Seiten der Blendenklappe in den Strömungskanal und dienen der Abnahme der durch die Blendenklappe erzeugten Druckdifferenz.

Auch bei einem Atemstromsensor gemäss der DE19617738C1 kann sich Flüssigkeit in dem Strömungskanal sammeln und es treten nach einer gewissen Gebrauchsdauer immer wieder Brüche im Scharnierbereich der Blendenklappe auf.

Die EP2275783A2 offenbart einen Atemstromsensor mit einem Strömungsrohr, das einen Strömungskanal und an dessen freien Enden Schlauchanschlüsse für Schläuche aufweist, mit einer Blendenklappe, die in dem Strömungskanal angeordnet ist und den Strömungskanal in einen ersten Strömungskanalteil und in einen zweiten Strömungskanalteil unterteilt. Die Blendenklappe weist einen Scharnierbereich auf, der von fünf parallel zueinander angeordneten Einschnitten gebildet ist. Zu beiden Seiten der Blendenklappe münden Anschlüssen in den Strömungskanal, die zur Abnahme der durch die Blendenklappe erzeugten Druckdifferenz dienen.

Weiter offenbart die EP2275783A2 einen Anschlagkörper mit zwei Anschlagsflächen. Der Anschlagskörper ist beim Scharnierbereich der Blendenklappe angeordnet und begrenzt die maximale Auslenkung derselben.

Aufgabe der vorliegenden Erfindung ist es somit, einen Atemstromsensor zu schaffen, der die vorgenannten Nachteile nicht aufweist und insbesondere eine lange Gebrauchsdauer gewährleistet.

Die Aufgabe wird durch die Merkmale des unabhängigen Anspruchs gelöst. Vorteilhafte Weiterbildungen sind in den Figuren und in den abhängigen Patentansprüchen dargelegt.

Gemäss der Erfindung weist ein Atemstromsensor ein Strömungsrohr, das einen Strömungskanal und an dessen freien Enden Schlauchanschlüsse für Schläuche aufweist, eine Blendenklappe, die in dem Strömungskanal angeordnet ist und den Strömungskanal in einen ersten Strömungskanalteil und in einen zweiten Strömungskanalteil unterteilt, wobei die Blendenklappe einen Scharnierbereich aufweist, der von einem ersten Einschnitt und zumindest einem zweiten Einschnitt gebildet ist, und Anschlüsse auf, die zu beiden Seiten der Blendenklappe in den Strömungskanal münden und zur Abnahme der durch die Blendenklappe erzeugten Druckdifferenz dienen, wobei der zumindest eine zweite Einschnitt des Scharnierbereichs der Blendenklappe H-förmig ausgebildet ist sowie jeweils benachbart zu dem zumindest einen zweiten Einschnitt je ein Seiteneinschnitt vorgesehen ist.

Diese Ausgestaltung der Einschnitte verhindert nicht nur eine Torsionsbewegung der Blendenklappe bei hohen Atemflüssen, sondern gewährleistet eine hohe Sicherheit gegen ein Brechen der Blendenklappe in deren Scharnierbereich. Die Blendenklappe weist vorteilhaft einen mehreckigen und/oder achssymmetrischen Querschnitt auf.

Bevorzugt weisen die Enden zumindest des zumindest einen zweiten Einschnitts Schrägen oder Radien auf, womit Spannungsspitzen beim Auslenken der Blendenklappe in diesen Bereichen der Einschnitte reduziert und somit die Gebrauchstauglichkeit der Blendenklappe und somit des gesamten Atemstromsensors verbessert wird. Vorteilhaft sind alle Enden der Einschnitte in der Blendenklappe mit Schrägen oder Radien versehen.

Bevorzugt weist ein Atemstromsensor ein Strömungsrohr, das einen Strömungskanal und an dessen freien Enden Schlauchanschlüsse für Schläuche aufweist, eine Blendenklappe, die in dem Strömungskanal angeordnet ist und den Strömungskanal in einen ersten Strömungskanalteil und in einen zweiten Strömungskanalteil unterteilt, wobei die Blendenklappe einen Scharnierbereich und einen dem Scharnierbereich gegenüberliegenden freien Endbereich aufweist, und Anschlüsse auf, die zu beiden Seiten der Blendenklappe in den Strömungskanal münden und zur Abnahme der durch die Blendenklappe erzeugten Druckdifferenz dienen, wobei zumindest ein Anschlagabschnitt für den freien Endbereich der Blendenklappe im Strömungskanal zur Begrenzung der Auslenkung der Blendenklappe vorgesehen ist.

Der zumindest eine Anschlagabschnitt limitiert die maximale Auslenkung der Blendenklappe, womit diese und somit der Atemstromsensor eine gegenüber den aus dem relevanten Stand der Technik bekannten Lösungen höhere Gebrauchstauglichkeit aufweist.

Vorteilhaft ist bei dem Anschlagabschnitt eine Entwässerungseinrichtung für die im Atemstromsensor anfallende Flüssigkeit vorgesehen, z. B. in Form von zumindest einer Abflussrinne oder einer Sammelwanne.

Vorzugsweise sind zwei, einander gegenüberliegende Anschlagabschnitte im Strömungskanal zur Begrenzung der Auslenkung der Blendenklappe in beide Richtungen vorgesehen, womit die Gebrauchstauglichkeit des Atemstromsensors zusätzlich verbessert wird, da die Blendenklappe nur über den durch den Abstand der Anschlagabschnitte zueinander definierten Bereich verschwenken kann.

Vorteilhaft ist zwischen den beiden Anschlagabschnitten eine Entwässerungseinrichtung für die im Atemstromsensor anfallende Flüssigkeit vorgesehen, z. B. in Form einer Sammelwanne.

Ein Atemstromsensor weist ein Strömungsrohr, das einen Strömungskanal und an dessen freien Enden Schlauchanschlüsse für Schläuche aufweist, eine Blendenklappe, die in dem Strömungskanal angeordnet ist und den Strömungskanal in einen ersten Strömungskanalteil und in einen zweiten Strömungskanalteil unterteilt, und Anschlüsse auf, die zu beiden Seiten der Blendenklappe in den Strömungskanal münden und zur Abnahme der durch die Blendenklappe erzeugten Druckdifferenz dienen, wobei eine Entwässerungseinrichtung in dem Strömungskanal vorgesehen ist.

Über die Entwässerungseinrichtung fliesst sich im Strömungskanal ansammelnde Feuchtigkeit beispielsweise ab oder diese kann in der Entwässerungseinrichtung gesammelt werden. Alternativ oder zusätzlich zu einer Ableitung der Flüssigkeit wird der maximale Stand der Flüssigkeit im Sensor durch die Entwässerungseinrichtung in dem Strömungskanal limitiert. Die Entwässerungseinrichtung in dem Strömungskanal gewährleistet somit, dass die im Atemstromsensor befindliche Flüssigkeit die Auslenkung beziehungsweise die Bewegung der Blendenklappe nicht beeinträchtigt und somit die im Atemstromsensor befindliche Flüssigkeit nicht zu verfälschten Messergebnissen führt.

Vorteilhaft umfasst die Entwässerungseinrichtung zumindest eine Abflussrille, in welcher sich Flüssigkeit ansammeln und/oder abfliessen kann. Vorteilhaft erstreckt sich die zumindest eine Abflussrille in Durchströmrichtung des Atemstromsensors beziehungsweise dessen Strömungskanals, womit eine kumulierte Ansammlung von Flüssigkeit im Bereich der Blendenklappe in bevorzugter Weise verhindert wird.

Die zumindest eine Abflussrille ist vorteilhaft kanalförmig ausgebildet und weist beispielsweise einen rechteckigen, U-förmigen und/oder V-förmigen Querschnitt auf.

Vorteilhaft umfasst die Entwässerungseinrichtung mehrere Abflussrillen, so dass trotz Bereitstellung eines ausreichend grossen Volumens zur Aufnahme von Flüssigkeit zur Verfügung steht, aber die Wandung des Strömungskanals nicht übermässig im Bereich der Abflussrillen geschwächt wird.

Vorteilhaft sind die Abflussrillen in Umfangsrichtung beabstandet zueinander angeordnet, so dass diese für eine Sammlung beziehungsweise Ableitung oder Abführung der Flüssigkeit optimal angeordnet sind. Auch bei einer Drehung des Atemstromsensors um seine Durchströmachse ist somit zumindest eine Abflussrille zur Sammlung beziehungsweise zur Abführung der Flüssigkeit vorhanden.

Vorteilhaft sind die mehrere Abflussrillen entlang einem Bereich des Innenumfangs des Strömungskanals angeordnet, der sich maximal über die Hälfte des gesamten Innenumfangs des Strömungskanals erstreckt. Bezogen auf den Querschnitt des Strömungskanals erstreckt sich der Bereich mit den Abflussrillen über maximal 180° eines Vollkreises mit 360°. Mit dieser Anordnung der mehreren Abflussrillen ist bei einer Drehung des Atemstromsensors um seine Durchströmachse im üblichen Anwendungsfall immer zumindest eine Abflussrille zur Sammlung beziehungsweise zur Abführung der Flüssigkeit vorhanden.

Vorteilhaft sind die Abstände der Abflussrillen zueinander entlang des Bereichs des Innenumfangs unterschiedlich, was eine vorteilhafte Sammlung beziehungsweise Abführung der Flüssigkeit im Atemstromsensor ermöglicht.

Vorteilhaft ist der Winkelbereich der äussersten Abflussrillen zu den von diesen benachbarten Abflussrillen grösser, als von den inneren Abflussrillen zueinander. Üblicherweise dreht sich der Atemstromsensor während der Beatmung beziehungsweise während der Anästhesie nur über einen kleinen Winkelbereich um die Durchströmachse. Infolge der näheren Anordnung der inneren Abflussrinnen zueinander steht bei einer üblichen Anwendung des Atemstromsensors und bezogen auf die Schwerkraft ein ausreichend grosses Volumen zur Aufnahme beziehungsweise Ableitung der Flüssigkeit zur Verfügung.

Vorteilhaft verläuft die zumindest eine Abflussrille ausgehend von der Blendenklappe in eine Richtung des entsprechenden freien Endes des Strömungsrohres entlang einer Bogenlinie, so dass durch die Schwerkraft eine vorteilhafte Ableitung oder Abführung der Flüssigkeit gegeben ist. Alternativ verläuft die zumindest eine Abflussrille ausgehend von der Blendenklappe in eine Richtung des entsprechenden freien Endes des Strömungsrohres entlang einer Schräge, die in einem Winkel zur Durchströmachse des Strömungskanals angeordnet ist.

Vorteilhaft ist die Entwässerungseinrichtung von zumindest einem Strömungskanalabschnitt gebildet ist, dessen Querschnitt sich ausgehend von der Blendenklappe in eine Richtung des entsprechenden freien Endes des Strömungsrohres vergrössert, so dass durch die Schwerkraft eine Ableitung oder Abführung der Flüssigkeit gegeben ist. Vorteilhaft vergrössert sich der Strömungskanalabschnitt über seinen gesamten Umfang, so dass die relative Lage des Atemstromsensors in Bezug auf die Durchströmachse des Strömungskanals keinen Einfluss hat.

Vorteilhaft ist der sich vergrössernde Strömungskanalabschnitt trompetenförmig ausgebildet, was eine einfache Herstellung bei gleichzeitig vorteilhafter Ableitung oder Abführung der Flüssigkeit ermöglicht. Alternativ ist der sich vergrössernde Strömungskanalabschnitt konisch ausgebildet.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind. Dabei können die in den Ansprüchen und in der Beschreibung erwähnten Merkmale jeweils einzeln für sich oder in beliebiger Kombination erfindungswesentlich sein.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei:
- Fig. 1: einen erfindungsgemässen Atemstromsensor in einer perspektivischen Ansicht,
- Fig. 2: den Atemstromsensor gem. Fig. 1 in einer Explosionsdarstellung,
- Fig. 3: einen schematischen Längsschnitt durch einen Atemstromsensor,
- Fig. 4A-B: jeweils einen den Atemstromsensor gem. Fig. 3 im Querschnitt,
- Fig. 5: einen schematischen Längsschnitt durch eine Variante des Atemstromsensors gem. Fig. 3,
- Fig. 6: einen schematischen Längsschnitt durch eine weitere Variante des Atemstromsensors gem. Fig. 3,
- Fig. 7: einen Atemstromsensor in einem Detailschnitt,
- Fig. 8: die Blendenklappe eines erfindungsgemässen Atemstromsensors gem. Fig. 1 in einer Ansicht, und
- Fig. 9: das Scharnier der Blendenklappe gem. Fig. 8 in einer Detailansicht.

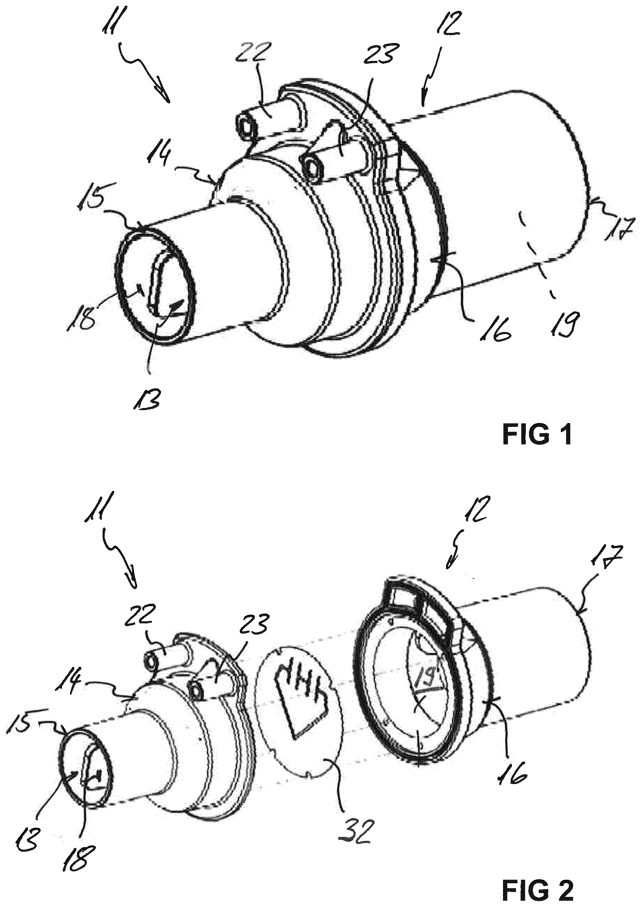

Der in den Figuren 1 und 2 dargestellte erfindungsgemässe Atemstromsensor 11 weist ein Strömungsrohr 12, eine Blendenklappe 32 und Anschlüsse 22 und 23 auf. Das zylindrische Strömungsrohr 12 weist einen Strömungskanal 13 sowie an dessen freien Enden 15 bzw. 17 Schlauchanschlüsse für Schläuche auf.

Das Strömungsrohr 12 ist zweiteilig (Strömungsrohrteil 14 und Strömungsrohrteil 16) ausgebildet, wobei die Blendenklappe 32 zwischen den Strömungsrohrteilen 14 und 16 im Strömungskanal 13 angeordnet ist. Die Blendenklappe 32 unterteilt den Strömungskanal 13 in einen ersten Strömungskanalteil 18 und in einen zweiten Strömungskanalteil 19. Im Bereich, in welchen die Blendenklappe 32 im zusammengeführten Zustand der Strömungsrohrteile 14 und 16 zu liegen kommt, weisen die Strömungsrohrteile 14 und 16 jeweils einen grösseren Durchfluss-Querschnitt auf.

Der Anschluss 22 mündet in den Strömungskanal 13 im ersten Strömungskanalteil 18 und der Anschluss 23 mündet in den Strömungskanal 13 im zweiten Strömungskanalteil 19. Die Anschlüsse 22 und 23 münden somit zu beiden Seiten der Blendenklappe 32 in den Strömungskanal 13 und dienen zur Abnahme der durch die Blendenklappe 32 erzeugten Druckdifferenz. In dem Strömungskanal 13 ist eine Entwässerungseinrichtung 41 vorgesehen.

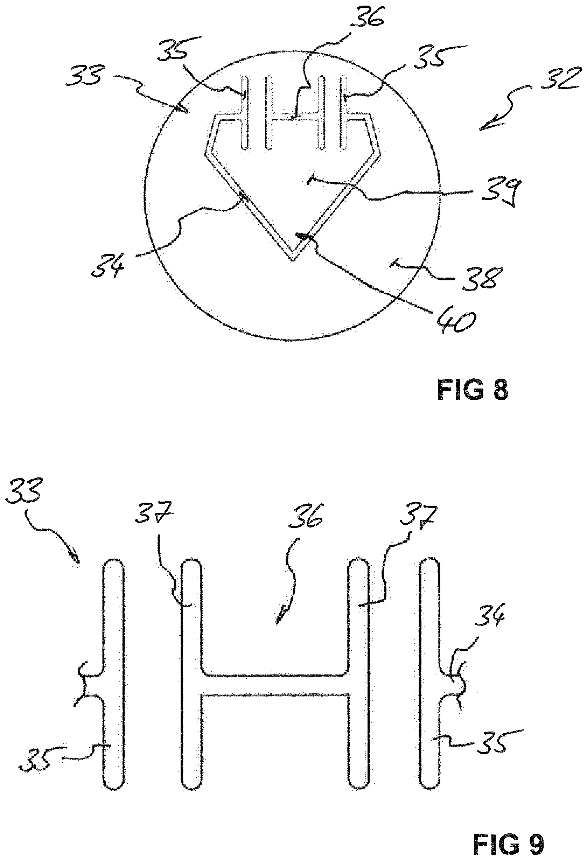

Die in den Figuren 8 und 9 dargestellte Blendenklappe 32 des erfindungsgemässen Atemstromsensor 11 weist hier einen kreisförmigen und scheibenförmigen Grundkörper mit einem im eingebauten Zustand festen Abschnitt 38 und einem beweglichen Klappenabschnitt 39. Der Klappenabschnitt 39 ist über einen Scharnierbereich 33 mit dem festen Abschnitt 38 verbunden und durch einen ersten Einschnitt 34 von diesem getrennt. Der Klappenabschnitt 39 weist einen dem Scharnierbereich 33 gegenüberliegenden freien Endbereich 40 auf. Der Scharnierbereich 33 ist von dem ersten Einschnitt 34 und einem zweiten Einschnitt 36 gebildet. Der zweite Einschnitt 36 ist H-förmig ausgebildet, wobei parallel beabstandet zu den Flanschen 37 des zweiten Einschnitts 36 jeweils noch ein Seiteneinschnitt 35 vorgesehen ist, in denen der erste Einschnitt 34 beidseitig mündet. Alle Enden des zweiten Einschnitts 36 und der Seiteneinschnitte 35 weisen Radien auf (siehe Fig. 9). Alternativ können diese Enden auch Schrägen aufweisen.

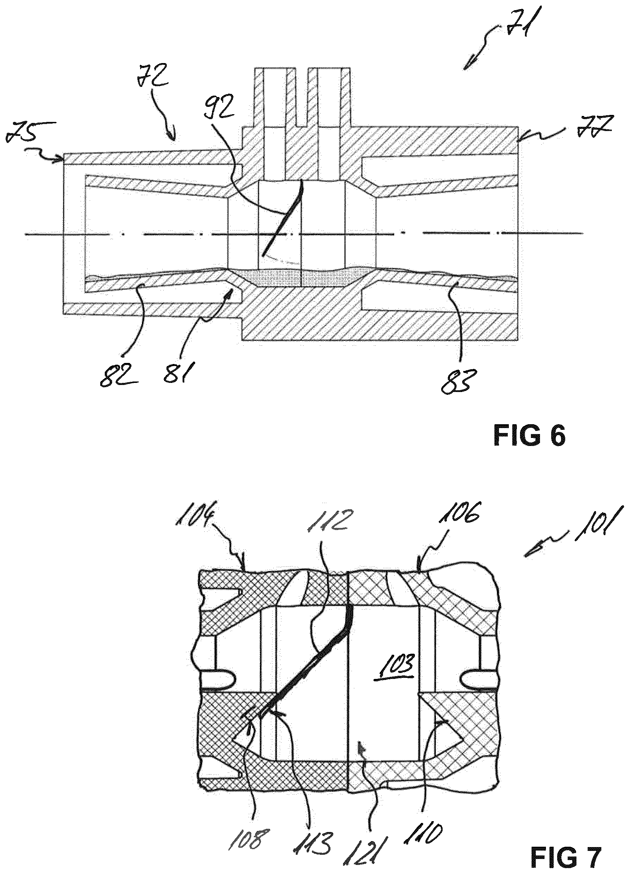

Der in Figur 7 nur ausschnittsweise gezeigte erfindungsgemässe Atemstromsensor 101 weist im Strömungsrohrteil 104 einen Anschlagabschnitt 108 und im Strömungsrohrteil 106 einen Anschlagabschnitt 110 auf, an welchen der freie Endbereich 113 der Blendenklappe 112 in Anlage kommen kann. Die einander gegenüberliegende Anschlagabschnitte 108 und 110 im Strömungskanal 103 begrenzen die maximale Auslenkung der Blendenklappe 112 in beide Richtungen. Zwischen den Anschlagabschnitten 108 und 110 ist eine Entwässerungseinrichtung 121 vorgesehen, die zur Aufnahme von Flüssigkeit wannenförmig ausgebildet ist. Zumindest ein solcher Anschlagabschnitt kann auch in dem Atemstromsensor 11, 51 oder 71 vorgesehen werden, wobei vorteilhaft beispielsweise durch die Anordnung entsprechender Ausnehmungen oder Einschnitte in dem Anschlagabschnitt der Abfluss der anfallenden Flüssigkeit gewährleistet wird.

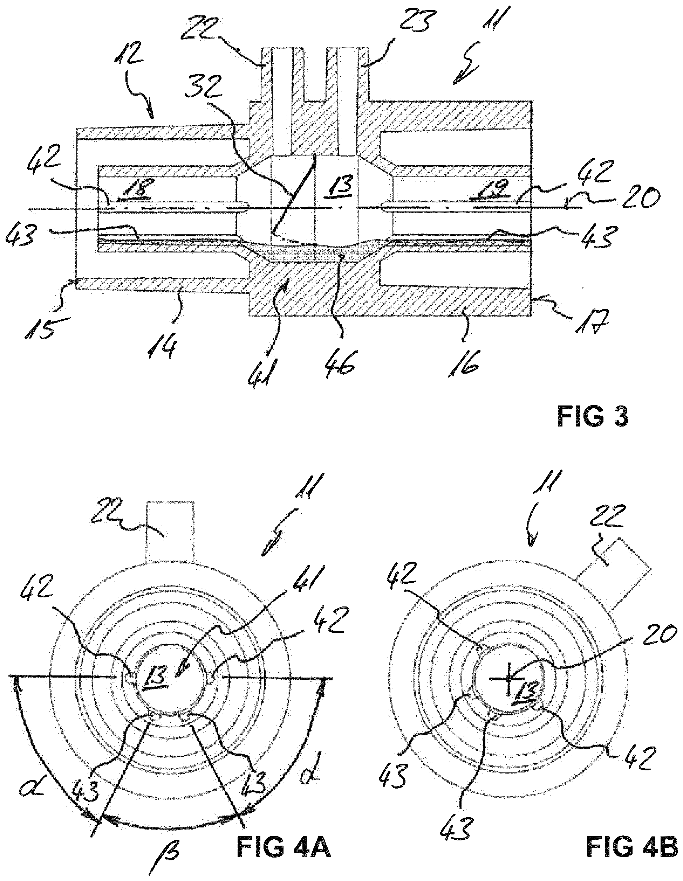

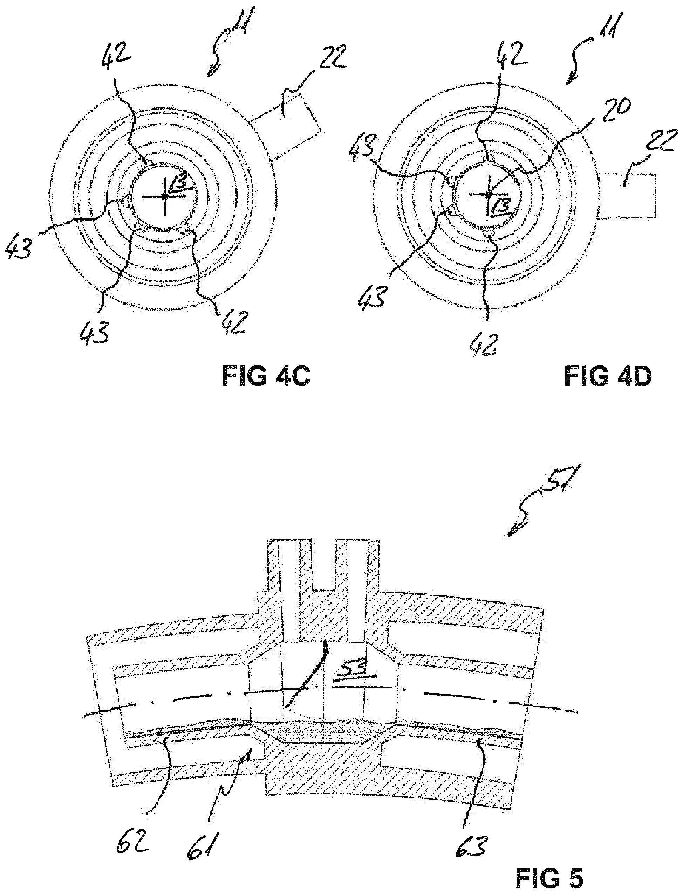

In der in den Figuren 3 und 4A bis 4D schematisch vereinfachten Darstellung umfasst die Entwässerungseinrichtung 41 mehrere Abflussrillen 42 bzw. 43, die umfänglich beabstandet zueinander angeordnet sind. Die im Atemstromsensor 11 anfallende Flüssigkeit 46 wird in den Abflussrillen 42 und 43 gesammelt und gegebenenfalls in diesen abgeleitet. Die Abflussrillen 42 und 43 sind derart ausgebildet, dass die Bewegung der Blendenklappe 32 nicht durch diese beeinträchtigt wird.

Die Abflussrillen 42 und 43 sind entlang eines Bereichs des Innenumfangs des Strömungskanals 13 angeordnet, welcher sich über die Hälfte des gesamten Innenumfangs des Strömungskanals 13 erstreckt (siehe Figur 4A; 2xα + β). Der Abstand (Winkelmass α) der Abflussrillen 42 zu den Abflussrillen 43 ist grösser als der Abstand (Winkelmass β) der Abflussrillen 43 zueinander. Eine derartige Anordnung der Abflussrillen 42 und 43 gewährleistet, dass mittels der Entwässerungseinrichtung 41 auch eine ausreichende Messgenauigkeit mit dem Atemstromsensor 11 erreicht wird, wenn dieser um seine Durchströmachse 20 bereichsweise verdreht wird (siehe Figuren 4A bis 4D). Die Durchströmachse 20 entspricht der Mittelachse des Strömungskanals 13.

Einzelne oder alle Abflussrillen 42 und/oder 43 können ausgehend von der Blendenklappe 32 in eine Richtung des entsprechenden freien Endes 15 bzw. 17 des Strömungsrohres 12 entlang einer Bogenlinie verlaufen. Alternativ können einzelne oder alle Abflussrillen 42 und/oder 43 ausgehend von der Blendenklappe 32 in eine Richtung des entsprechenden freien Endes 15 bzw. 17 des Strömungsrohres 12 entlang einer Schräge verlaufen, die in einem Winkel zur Durchströmachse 20 des Strömungskanals 13 angeordnet ist.

Bei dem in der Figur 5 schematisch vereinfacht dargestellten Ausführungsbeispiel ist die Entwässerungseinrichtung 61 des Atemstromsensor 51 von Strömungskanalabschnitten 62 und 63 gebildet, die jeweils bogenförmig verlaufen. Auch der Strömungskanal 53 verläuft bogenförmig.

Bei dem in der Figur 6 schematisch vereinfacht dargestellten Ausführungsbeispiel ist die Entwässerungseinrichtung 81 des Atemstromsensor 71 von Strömungskanalabschnitten 82 und 83 gebildet, deren Querschnitte sich ausgehend von der Blendenklappe 92 in eine Richtung des entsprechenden freien Endes 75 bzw. 77 des Strömungsrohres 72 vergrössert. Die Strömungskanalabschnitte 82 bzw. 83 sind jeweils konisch ausgebildet. Alternativ könnte zumindest einer der Strömungskanalabschnitte 82 oder 83 auch trompetenförmig ausgebildet sein.

**Bezugszeichenliste**

| | | | |
|---|---|---|---|
| 11 | Atemstromsensor | 51 | Atemstromsensor |
| 12 | Strömungsrohr | 53 | Strömungskanal |
| 13 | Strömungskanal | | |
| 14 | Strömungsrohrteil | 61 | Entwässerungseinrichtung |
| 15 | freies Ende v. 14 | 62 | Strömungskanalabschnitt |
| 16 | Strömungsrohrteil | 63 | Strömungskanalabschnitt |
| 17 | freies Ende v. 16 | | |
| 18 | 1. Strömungskanalteil | | |
| 19 | 2. Strömungskanalteil | 71 | Atemstromsensor |
| 20 | Durchströmachse | 72 | Strömungsrohr |
| | | 75 | freies Ende |
| 22 | Anschluss | 77 | freies Ende |
| 23 | Anschluss | 81 | Entwässerungseinrichtung |
| 32 | Blendenklappe | 82 | Strömungskanalabschnitt |
| 33 | Scharnierbereich | 83 | Strömungskanalabschnitt |
| 34 | 1. Einschnitt | | |
| 35 | Seiteneinschnitt | 92 | Blendenklappe |
| 36 | 2. Einschnitt | | |
| 37 | Flansch v. 36 | | |
| 38 | fester Abschnitt v. 32 | 101 | Atemstromsensor |
| 39 | Klappenabschnitt v. 32 | 103 | Strömungskanal |
| 40 | freier Endbereich v. 39 | 104 | Strömungsrohrteil |
| 41 | Entwässerungseinrichtung | 106 | Strömungsrohrteil |
| 42 | Abflussrille | 108 | Anschlagabschnitt |
| 43 | Abflussrille | 110 | Anschlagabschnitt |
| α | Winkel zw. 42 und 43 | 112 | Blendenklappe |
| β | Winkel zw. 43 und 43 | 113 | freier Endbereich v. 112 |
| | | 121 | Entwässerungseinrichtung |

## Patentansprüche

1. Atemstromsensor mit einem Strömungsrohr (12), das einen Strömungskanal (13) und an dessen freien Enden (15, 17) Schlauchanschlüsse für Schläuche aufweist, mit einer Blendenklappe (32), die in dem Strömungskanal (13) angeordnet ist und den Strömungskanal (13) in einen ersten Strömungskanalteil (18) und in einen zweiten Strömungskanalteil (19) unterteilt, wobei die Blendenklappe (32) einen Scharnierbereich (33) aufweist, der von einem ersten Einschnitt (34) und zumindest einem zweiten Einschnitt (36) gebildet ist, und mit Anschlüssen (22, 23), die zu beiden Seiten der Blendenklappe (32) in den Strömungskanal (13) münden und zur Abnahme der durch die Blendenklappe(32) erzeugten Druckdifferenz dienen, **dadurch gekennzeichnet, dass** der zumindest eine zweite Einschnitt (36) des Scharnierbereichs (33) der Blendenklappe (32) H-förmig ausgebildet ist sowie jeweils benachbart zu dem zumindest einen zweiten Einschnitt (36) je ein Seiteneinschnitt (35) vorgesehen ist.

2. Atemstromsensor nach Anspruch 1, **dadurch gekennzeichnet, dass** die die Enden zumindest des zumindest einen zweiten Einschnitts (36) Schrägen oder Radien aufweisen.

3. Atemstromsensor nach Anspruch 1 oder 2, **dadurch gekennzeichnet, dass** die Blendenklappe (112) einen dem Scharnierbereich gegenüberliegenden freien Endbereich (113) aufweist, wobei ein Anschlagabschnitt (108, 110) für den freien Endbereich (113) der Blendenklappe (112) im Strömungskanal (103) zur Begrenzung der Auslenkung der Blendenklappe (112) vorgesehen ist.

4. Atemstromsensor nach Anspruch 3, **dadurch gekennzeichnet, dass** zwei, einander gegenüberliegende Anschlagabschnitte (108, 110) im Strömungskanal (103) zur Begrenzung der Auslenkung der Blendenklappe (112) vorgesehen sind.

## Claims

1. A respiratory flow sensor with a flow pipe (12) which has a flow channel (13) and hose connections for hoses on the free ends (15, 17) thereof, with a cover flap (32) which is disposed in the flow channel (13) and which divides the flow channel (13) into a first flow channel section (18) and into a second flow channel section (19), wherein the cover flap (32) has a hinge region (33) which is formed by a first incision (34) and at least one second incision (36), and with connections (22, 23) which lead into the flow channel (13) on both sides of the cover flap (32) and which serve to reduce the pressure differential generated by the cover flap (32), **characterized in that** the at least one second incision (36) of the hinge region (33) of the cover flap (32) is H-shaped in configuration, and in addition, a lateral incision (35) is provided respectively adjacent to each at least one second incision (36).

2. The respiratory flow sensor as claimed in claim 1, **characterized in that** the ends of at least the at least one second incision (36) are inclined or are radii.

3. The respiratory flow sensor as claimed in claim 1 or claim 2, **characterized in that** the cover flap (112) has a free end region (113) which is opposite to the hinge region, wherein a stop section (108, 110) for the free end region (113) of the cover flap (112) is provided in the flow channel (103) in order to limit the deflection of the cover flap (112).

4. The respiratory flow sensor as claimed in claim 3, **characterized in that** two stop sections (108, 110) which are positioned opposite to one another are provided in the flow channel (103) in order to limit the deflection of the cover flap (112).

## Revendications

1. Capteur de flux respiratoire, pourvu d'un tube d'écoulement (12), qui comporte un canal d'écoulement (13) et sur ses extrémités libres (15, 17) des raccords à flexibles, pour des flexibles, pourvu d'un clapet formant diaphragme (32), qui est placé dans le canal d'écoulement (13) et qui divise le canal d'écoulement (13) en une première partie de canal d'écoulement (18) et en une deuxième partie de canal d'écoulement (19), le clapet formant diaphragme (32) comportant une zone de charnière (33), qui est formée d'une première incision (34) et d'au moins une deuxième incision (36) et pourvu de raccords (22, 23), qui vers les deux côtés du clapet formant diaphragme (32) débouchent dans le canal d'écoulement (13) et qui servent à reprendre la pression différentielle générée par le clapet formant diaphragme (32), **caractérisé en ce que** l'au moins une deuxième incision (36) de la zone de charnière (33) du clapet formant diaphragme (32) est conçue en forme de H et **en ce qu'**au voisinage de l'au moins une deuxième incision (36) est prévue respectivement chaque fois une incision latérale (35).

2. Capteur de flux respiratoire selon la revendication 1, **caractérisé en ce que** les extrémités d'au moins l'au moins une deuxième incision (36) comportent des inclinaisons ou des rayons.

3. Capteur de flux respiratoire selon la revendication 1 ou 2, **caractérisé en ce que** le clapet formant diaphragme (112) comporte une zone d'extrémité (113) opposée à la zone de charnière, un segment formant butée (108, 110) pour la zone d'extrémité (113) libre du clapet formant diaphragme (112) étant prévu dans le canal d'écoulement (103) pour limiter la déviation du clapet formant diaphragme (112).

4. Capteur de flux respiratoire selon la revendication 3, **caractérisé en ce que** deux segments formant butée (108, 110) opposés sont prévus dans le canal d'écoulement (103) pour limiter la déviation du clapet formant diaphragme (112).
